# EUROPEAN PATENT SPECIFICATION

(11) **EP 0 381 483 B1**
(45) Date of publication and mention of the grant of the patent: **22.06.1994**
(21) Application number: 90301026.2
(22) Date of filing: 31.01.1990
(51) Int. Cl.: C07H 15/04, A23L 1/236

(54) **Crystalline lacticol trihydrate, a solid crystalline mixture containing it and a process for preparing them**
Kristallines Lactittrihydrat, dieses enthaltende feste kristalline Mischung und ein Verfahren zu deren Herstellung
Trihydrate de lactitol cristallin, mélange cristallin solide le contenant et procédé pour leur préparation

(30) Priority: 31.01.1989 JP 19451/89
(43) Date of publication of application: 08.08.1990
(73) Proprietor: TOWA CHEMICAL INDUSTRY CO., LTD., Chuo-ku, Tokyo 104 (JP)
(72) Inventor: Kawashima, Shigeru, Fujinomiya-shi, Shizuoka, 418 (JP); Ide, Hiroshi, Fuji-shi, Shizuoka, 417 (JP); Kato, kazuaki, Kitakatsushika-gun, Saitama, 342 (JP); Magara, Mitsuo, Numazu-shi, Shizuoka, 410-03 (JP); Ishii, Yoshibumi, Fuji-shi, Shizuoka, 417 (JP)
(74) Representative: Holmes, Michael John

(56) References cited:
- EP-A- 0 039 981
- DE-B- 2 133 428
- US-A- 3 957 976
- US-A- 3 973 050
- JOURNAL OF AGRICULTURAL AND FOOD CHEMISTRY, vol. 27, 1979 J.A. VAN VELTHUIJSEN: "Food Additives derived from Lactose: Lactitol and Lactitol Palmitate " pages 680-686.

## Description

The present invention relates to a crystalline lactitol trihydrate, a solid crystalline mixture containing it and a process for preparing them.

Lactitol, or 4-β-D-galactopyranosyl-D-sorbitol, is a product of lactose where the glucose moiety has been reduced to sorbitol.

Lactitol and conventional methods for its production are described in J. Agricultural and Food Chemistry., 27, 4, 680-686 (1979), in which a starting material comprising a 30-40% by weight aqueous lactose solution is hydrogenated under a hydrogen pressure of 40 atm at 100°C in the presence of Raney nickel. The catalyst is then settled and removed by filtration, and a product obtained after purification, for example with ion exchange resin or activated carbon.

Lactitol thus obtained has a relative sweetness of 36% (100% being the sweetness of a 5% aqueous sucrose solution - the lactitol solution having the same concentration) which is lower than that of sorbitol (relative sweetness 65%) or xylitol (relative sweetness 96%).

West-German Patent (MAIZENA, 1974) discloses that lactitol is hydrolysed by α-glucosidase (maltase) at a substantially slower rate than lactose or maltose.

For instance, lactose is hydrolysed almost completely by β-glactosidase within 45 min, while only 10-15% lactitol is hydrolysed within 45 min.

Thus, the absorbence and utilization of lactitol in the digestive system, and the fermentation of lactitol by intrabuccal bacteria is low. It is therefore suitable as a sweetening source for low-calorie foods, dietary foods, low cariogenic foods, health foods or the like, which are ingested, for example, by diabetics, obese persons, those who are health conscious or those wishing to avoid dental caries.

Lactitol has a lower hygroscopicity than sorbitol, glycerol, xylitol or the like, and since an aldehyde group has been reduced this results in increased stability to heat or alkali, allowing lactitol to be used advantageously for a variety of foods.

Lactitol crystals are well described in the literature. For example, crystalline lactitol dihydrate having a melting point of 76-78°C and a specific rotation of +12.2°C, and anhydrous crystalline lactitol having a melting point of 146°C and a specific rotation of +14°C are described in Comptes Rendus Hebdomadaires des Séances de l'Académie des Sciences, 170, 47-50 (1920) and J. Am. Chem. Soc., 74, 1105 (1952).

Moreover, crystalline lactitol monohydrate is described in J. Agricultural and Food Chemistry, 27, 4, 680-686 (1979), in which crystalline lactitol containing 1% of lactulitol (4-β-D-galactosyl-D-mannitol) and 3% of mannitol is disclosed.

European patent application EP-A-39981 describes crystalline lactitol monohydrate and crystalline lactitol dihydrate and the preparation thereof from aqueous solutions of lactitol.

However, conventional anhydrous crystalline lactitol, lactitol monohydrate, lactitol dihydrate and methods for producing them have a variety of disadvantages in production or application, and it has been desirable to reduce or eliminate these disadvantages.

Specifically, anhydrous crystalline lactitol as described in the prior art has the disadvantages that: (i) a relatively difficult drying process or other length procedures are required in production; (ii) the anhydrous lactitol produced is relatively hydroscopic and absorbs humidity from the atmosphere, leading to a deterioration in quality with time if a conventional packaging material or a container is used as employed for analogous sugar alcohols such as an anhydrous crystalline maltitol or the like; or (iii) the anhydrous lactitol has a high melting point, so that a high temperature is required, when it is used as a molten dispersant for other powdered materials. This may lead to the other components decomposing.

The crystalline lactitol monohydrate form which has hitherto been known in the art has the drawback that it contains impurities such as mannitol, lactulitol or the like. Furthermore, it has a bitter taste, so that it is not suitable for use in foodstuffs. It has a high melting point, in the range of 94-97°C, and thus feels rough at a temperature of ca. 60°C and it is difficult to reproducibly control the quality of the crystals obtained.

Moreover, there are also disadvantages to the dihydrate crystal form of lactitol, for example, that it has a melting point of 76-78°C, which is too high for the crystals to dissolve when powdered onto warm food and leads to a rough mouth feel, producing an undesirable sensation on eating. Hence, its use in foodstuffs is restricted.

Thus, improvements of the aforementioned problems have been desired.

We have investigated the physicochemical properties of lactitol over a long period and have conducted intense research for crystalline lactitol having improved properties.

As a result thereof, we have discovered, that crystallization of lactitol from an aqueous lactitol solution leads to the formation of a crystalline trihydrate of lactitol having improved properties.

As a result thereof, we have discovered, that crystallization of lactitol from an aqueous lactitol solution leads to the formation of a crystalline trihydrate of lactitol which has not been previously described in the literature. This has excellent properties for the above applications including formation of a solid crystalline mixture containing it and a process for producing them, and we have succeeded in the advantageous production of the crystalline trihydrate of lactitol and the solid crystalline mixtures containing it on an industrial scale.

Thus, viewed from one aspect, the present invention comprises the crystalline trihydrate of lactitol which is represented by the molecular formula C₁₂H₂₄O₁₁.3H₂O and has a melting point of 52-56°C. The invention also provides a solid crystalline mixture containing the said trihydrate of lactitol and processes for producing the crystalline trihydrate of lactitol and a solid crystalline mixture containing it.

The physico-chemical properties of the crystalline trihydrate of lactitol according to the present invention is detailed below;
(1)

| Elementary analysis | | | |
|---|---|---|---|
| Observed values: | C = 36.1%, | H = 7.6%, | O = 56.3% |
| Calculated values: | C = 36.2%, | H = 7.6%, | O = 56.2% |

(2) Molecular weight
398.4
(3) Melting point
52.0-56.4°C (measured by visual observation by heating with a programming rate of 0.2°C/min starting from a temperature below the melting point)
(4) Differential scanning calorimetry
Maximum endotherm = 58-65°C (measured at a programming rate of 10°C/min with a differential scanning calorimeter)
(5) Specific rotation
[α]$\frac{\text{20}}{\text{D}}$ = +12.35° (0.1 g in 1 cc of water)
(6) Ultraviolet absorption
No characteristic absorption was measured for an aqueous solution.
(7) Infrared absorption spectrum
A 10 mg portion of the powder of the trihydrate crystal of lactitol and 440 mg of dry KBr were mixed by agitation to make a transparent tablet (thickness, ca. 0.6 mm) for the measurement of infrared absorption spectrum. The results are shown in Table 1.

**Table 1**

| Wave length at which the absorption of infrared rays are observed |
|---|
| 3100-3500 (nm) |
| 2840-2920 |
| 1600-1650 |
| 1200-1430 |
| 1000-1100 |

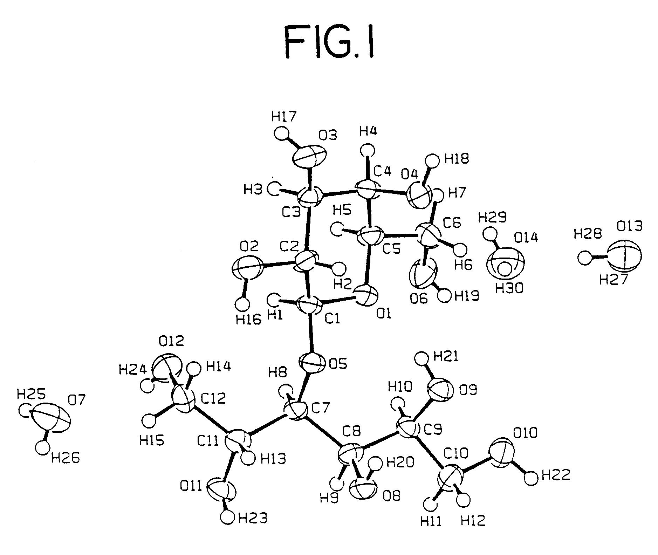

(8) Solubility
The solubility of crystalline trihydrate of lactitol in 100 g of water at 25°C is about 160 g.
(9) Physical properties, colour of the material
The crystals are colourless transparent, and fine crystals are a white powder. They have a sweetness of 35-40% in comparison to sucrose and are odourless. The crystals are not deliquescent and exhibit little hygroscopicity, thus only a weight increment that is within the range of error upon storage at a temperature of 30°C and a relative humidity of 72% for 24 hours is observed. After drying under a pressure of 720-740 mmHg at 80°C for 3 hours, a weight reduction of about 13.5% is observed.
(10) Solubility in a variety of solvents.
Easily soluble in water, 0.1 N NaOH and 0.1 N HCl; soluble in methanol;
sparingly solutble in ethanol; and
insoluble in chloroform and ethyl acetate.
(11) X-ray crystallographic structure analysis
As a result of X-ray crystallographic structure analysis with the use of a single crystal of the trihydrate of lactitol according to the present invention, it was found that the crystals belong to the rhombic group, and the unit lattice is composed of 4 lactitol molecules and 12 water molecules. The unit lattice has dimensions a = 10.152 Å, b = 21.3029 Å and c = 8.301 Å and a unit lattice volume of 1795 Å³. The crystal has a calculated density of 1.474 g/cm³ and a space group of P2₁2₁2₁. The crystal structure is illustrated in Fig. 1.

From the above-described results, it is judged that the crystalline trihydrate of lactitol of the present invention and the solid crystalline mixture containing it are novel and different from the prior art described in the literature.

Processes for producing a crystalline trihydrate of lactitol according to the present invention and the solid crystalline mixture containing it are described below.

A lactitol solution for crystallization used in the present invention may utilize lactitol obtained by any convenient process.

The process for crystallization of crystalline trihydrate of lactitol according to the present invention preferably comprises adding to a lactitol solution with a concentration of at least 60-85% by weight at 0°C a seed crystal having 0.01-20% by weight of solid content relative to the solution at a temperature of 0-30°C and cooling with slow agitation. A crystal mass can be thus formed.

An organic solvent such as ethanol or acetone can also be present in order to control the degree of supersaturation or the viscosity of the solution.

Thus, the crystalline trihydrate of lactitol according to the present invention or the solid crystalline mixture containing it, can be crystallized relatively easily by adding a seed crystal of either a crystalline trihydrate of lactitol or a solid crystalline mixture containing it to a supersaturated lactitol solution.

For the process of producing the crystalline trihydrate of lactitol or the solid crystalline mixture containing it from the resultant crystal mass, a process is required to allow collection of the product. When the crystal mass has little water content it can be simply solidified. When the crystal mass contains a large amount of water, it is also possible to collect separate trihydrate crystals of lactitol.

It is possible to use a variety of well-known methods such as a solid incorporating or solid separating methods, for example, by kneading, block grinding, fluid granulation, spray-drying or the like, as the process for producing crystalline trihydrate of lactitol.

Specifically, the solid separating method is usually a method for separating the crystal means into separate trihydrate crystals of lactitol and other solids by centrifugation. It is also possible, if necessary, according to the solid separating method to wash the crystals with a small amount of water, for example, by spraying cool water, and thus such a method is suited for the production of trihydrate crystals of lactitol having a higher purity and a melting point of 52-56°C.

Other methods such as kneading, block grinding, fluid granulation and spray-drying do not isolate the solid, and thus the solid crystalline mixture obtained may contain, in addition to trihydrate crystals of lactitol according to the present invention, a trace amount of the known dihydrate crystal form of lactitol or other sugar alcohols such as lactulitol, mannitol, sorbitol or the like as a component.

The kneading method - one of the solid incorporating methods - for example, comprises slowly cooling a lactitol solution having a water content of 15-40% in a kneader, adding a seed crystal having 5-50% by weight with a solid content corresponding to the solution at a temperature of or less than the melting point of the trihydrate crystal of lactitol, preferably 0-30°C. Alternatively, the mixture can be stirred, without the addition of the seed crystal, and the mixture formed into a variety of shapes or forms such as powder, granules, globes, rods, plates, cubes or the like to produce the powder or the moulded products of the solid crystalline mixture containing crystalline trihydrate of lactitol.

Crystalline trihydrate of lactitol or the solid crystalline mixture containing it according to the present invention have excellent properties compared with the conventional anhydrous lactitol or dihydrate crystals of lactitol.

For example, when compared with anhydrous lactitol, the trihydrate crystalline form is advantageous in several respects, for example, having a moderately low melting point, requiring relatively easy procedures for drying and exhibiting substantially no hygroscopicity so that a relatively cheap packaging material can be used.

When compared to the dihydrate crystal form of lactitol or the monohydrate crystal form of lactitol, the trihydrate crystal form of lactitol according to the present invention is advantageous in that it has an extremely low melting point of 52-56°C, so that it dissolves smoothly on eating and thus has a good sweetness and mouth feel and that it can be changed into syrup upon powdering onto warm food at a temperature of 50-70°C.

Thus, it can be used advantageously for foods, cosmetics, pharmaceutical products, chemical materials and the like.

The crystalline trihydrate of lactitol can be used, if required, in admixture with appropriate amounts of one or more of other sweeteners such as starch syrup solid, glucose, maltose, high fructose corn syrup, sucrose, honey, maple syrup, sorbitol, dihydrocharcone, stevioside, α-glycosyl stevioside, glycyrrhizin, saccharin, aspartame, glycine, alanine or the like. It can also be used in admixture with fillers such as dextrin, starch, polydextrose, lactose or the like.

Also, the powder of crystalline trihydrate of lactitol according to the present invention and the solid crystalline mixture containing it can optionally be used intact or, if required, mixed with fillers, vehicles, binders, disintegrating agents or the like and then formed into shapes such as granules, globes, tablets, rods, plates, cubes or the like.

Since crystalline trihydrate of lactitol according to the present invention and the solid crystalline mixture containing it are similar to other lactitols or lactitol crystal forms in being poorly digested or absorbed by the digestive system, it is possible to reduce the calorific value of foods and drinks which are sweetened by the addition of crystalline trihydrate lactitol or the solid crystalline mixture containing it according to the present invention.

Thus, crystalline trihydrate of lactitol according to present invention and the solid crystalline mixture containing it can be utilized as a low-calorie sweetener for diabetics, obese persons and those whose calorific-intake is restricted or for giving a sweet flavour to low-calorie foods such as slimming aids, health foods or dietaries.

Moreover, crystalline trihydrate of lactitol according to present invention and the solid crystalline mixture containing it are selectively utilized by Lactobacillus bifidus and are hardly fermented by cariogenic bacteria, so that they can be also used as a Lactobacillus bifidus growth activator or as a low-cariogenic sweetener.

Thus, crystalline trihydrate of lactitol according to present invention and the solid crystalline mixture containing it are suitable for the sweetening of low-cariogenic foods and drinks such as confectioneries, for example, chewing gum, chocolate, biscuits, cookies, caramel and candy; and soft drinks such as, for example, cola drinks, cider, juice, coffee and yoghurt drinks. They are also suitable for the sweetening of cosmetics and drugs for preventing caries such as gargles and toothpaste.

The sweetness of crystalline trihydrate of lactitol according to present invention and the solid crystalline mixture containing it are compatible with a variety of materials which have other tastes such as sourness, saltiness, astringency, deliciousness or bitterness, and have a high resistance to acid or heat, so that they can be used optionally not only for the above-mentioned special cases but also for the sweetening or improvement of the taste or the quality of usual or everyday foods and drinks.

For instance, they can be used optionally in various seasonings such as soy sauce, soy sauce powder, soy sauce paste "MISO", soy sauce paste powder, unrefined soy "MOROMI", salted meat, a variety of fish flours, mayonnaise, dressing, vinegar, vinegar sauce flavoured with sake and soy, vinegar powder, extracts for Chinese-style foods, soup for tempura, soup for noodles, sauce, catchup, sauce for roast meat, curry roux, extract for stew, extract for soup, extract for stock, mixed seasoning, nucleic acid type seasoning, sweet sake "MIRIN", new type sweet sake, table sugar, coffee sugar, fondant or the like.

They can also be used optionally as sweeteners or agents for improving the tastes or qualities of usual foods and drinks in various Japanese-style confections such as crackers, rice cake cubes, millet-and-rice cakes, rice cakes, bun with a bean-jam filling, sweet rice jelly, bean jams, fine sweet paste, transparent sugar cube "KINGYOKU", jelly, castella, starch based candy and the like; confections such as bread, biscuits, crackers, cookies, pies, puddings, butter cream, custard cream, cream puffs, waffles, sponge cakes, doughnuts, chocolate, chewing gum, caramel, candy and the like; syrup preserves such as fruit preserved in syrup, crystal sugar-egg white concentrate preserved in syrup "KORIMITSU" and the like; pastes such as flour paste, peanut paste, fruit paste and the like; processed foods of fruits or vegetables such as jam, marmalade, preserves, bonbons and the like; processed crop foods such as bread, noodles, rice, artificial meat and the like; pickles such as sliced vegetable pickled in soy sauce, fresh radish pickles, sliced vegetable root pickles, pickled scallions and the like; pickle additives such as an additive for pickled radish, an additive for pickled white rape and the like; meat products such as ham, sausage and the like; fish products such as fish ham, fish sausage, boiled fish paste, cylindrical boiled fish paste, tempura and the like; various seasoned marine products such as seasoned sea urchin egg, salted cuttlefish guts, sliced dry cuttlefish, dried MIRIN-seasoned swellfish, sliced tangle with sour taste and the like; dried laver, foods boiled down in soy sauce made of materials such as edible wild plants, dried cuttlefish, small fish, shellfish or the like; everyday dishes such as cooked beans, potato salad, tangle roll and the like; bottled or canned foods such as dairy products, fish, meat, fruits or vegetables; alcoholic drinks such as synthetic wine, fruit wine, whisky and brandy, and the like; soft drinks such as coffee, cocoa, juice, carbonated drinks, lactic acid drinks and the like; premixed type powders such as pudding mix, pancake mix and the like; and convenience type foods and drinks such as juice, coffee, adzuki-bean soup with rice cake, soup and the like.

Crystalline trihydrate of lactitol according to present invention and the solid crystalline mixture containing it have sparing hygroscopicity and good fluidity, and thus they can also be advantageously used as an anti-adhesion agent or a slippage improver, for example, in the case of chewing gum, sliced tangle with sour taste or the like by covering the surfaces of these products with crystalline trihydrate of lactitol according to present invention or the solid crystalline mixture containing it.

They can also be used for improving the taste of pet foods or food for cattle, fowl, honey bees, silkworms, fish, dogs or cats, or for stimulating the growth of Lactobacillus bifidus from animal sources.

In this connection, a variety of forms such as solids, pastes, liquids or the like can optionally be used in taste improving agents, flavouring agents or quality improving agents of, for example, table luxuries, cosmetics or drugs, such as tobacco, toothpaste, lipstick, lipcream, drugs for internal administration, troche, cod-liver oil, oral refreshing agents, cachou, gargles and the like.

Crystalline trihydrate of lactitol according to present invention and the solid crystalline mixture containing it can be formed into any desirable shape, for example, cubes, fish, flowers or the like, by moistening and compression moulding under a low pressure in a manner similar to moulded sugar prepared from granulated sugar. Hence moulded sweeteners suitable for coffee, tea or the like can be prepared.

Furthermore, the moulded sweeteners can optionally incorporate a variety of saccharides or artificial sweeteners for increasing sweetness, or coloured with a variety of food dyes or be incorporated with a variety of flavouring agents.

It is also possible to form these flavours into a clathrate compound such as cyclodextrin or the like which can subsequently be incorporated into the moulded sweeteners.

Crystalline trihydrate of lactitol according to the present invention can easily be prepared into massive crystals, similarly to sucrose, and thus can be also used as a transparent or translucent non-hygroscopic sweetener in place of candy sugar or coffee sugar.

Moreover, crystalline trihydrate of lactitol and the solid crystalline mixture containing it can be incorporated with, for example, vitamins, antibiotics or microorganisms of the Lactobacillus genus and formed into a variety of shapes such as granules or tablets for use in a variety of applications.

Fig. 1 is an illustration of the X-ray crystallographic structure analysis of a trihydrate crystal of lactitol according to the present invention.

The present invention is further illustrated by the following, non-limiting examples.

In the examples, all percentages are by weight unless otherwise specified.

### Example 1

A 200 g portion of an aqueous lactitol solution having a purity of 99.9%and a concentration of 82% was placed in a stainless tray and cooled from a temperature of 50°C to 10°C over a period of 20 hours to give a variety of crystal masses. Among these crystal masses, the one in which needle crystals had been formed radially was selected and put on each of the melting point blocks which was controlled at a predetermined constant temperature to measure the melting point by observing whether it melted or not. The melting point was 54°C.

### Example 2

A 80 g portion of an aqueous lactitol solution having a purity of 99.9% and a concentration of 80% was placed in a laboplastomill, and 17 g of the crystalline material obtained by the method in Example 1 was added at 10°C. The mixture was kneaded at a rate of 40 rpm for 10 min to give 90 g of a crystalline mixture solid containing the crystalline trihydrate of lactitol of the present invention. The melting point of the product, measured by placing it on each of the melting point blocks which was controlled at a predetermined constant temperature, was found to be 52.2°C.

The product is substantially non-hydroscopic, melts at an appropriate temperature and can be easily prepared, so that it can be advantageously used as a sweetener or a quality improver of a variety of foods and drinks, cosmetics, drugs or the like, or as a raw material for chemical industry.

### Example 3

A 840 g portion of an aqueous lactitol solution having a concentration of 75.0% (lactitol purity, 99.8%) was cooled gradually starting from 80°C. 0.9 g of a seed crystal (product obtained by the method in Example 2) was added to the solution at 15°C, and the mixture was cooled with stirring for 20 hours to give a crystal mass.

The crystal mass was centrifuged to give about 340 g (wet weight) of a crystal and 497 g of a filtrate having a concentration of 67.0%.

The crystal mass thus obtained was dried at a temperature of 20°C for 18 hours by a cold air dehumidifying dryer to give the crystalline trihydrate of lactitol of the present invention.

The melting point measured in the same manner as in Example 1 was 55.8°C.

Elementary analysis gave the values of C = 36.1%, H = 7.6% and O = 56.3%, while the theoretical values were C = 36.2%, H = 7.6% and O = 56.2%.

As a result of the differential scanning calorimetry (referred to hereinafter as DSC), the maximum endotherm at a programming rate of 10°C/min was 63.0°C.

### Example 4

A 72 g portion of an aqueous lactitol solution having a purity of 99.9% and a concentration of 79% was placed in a laboplastomill, and 18 g of the crystalline mixture solid obtained by the method in Example 2 was added at 5°C. The mixture was kneaded at a rate of 40 rpm for about 3 min to give a solid crystalline mixture containing crystalline trihydrate of lactitol of the present invention.

As a result of measuring the melting point of the product in the same manner as described in Example 1, the melting point was found to be 53.1°C and the maximum endotherm by DSC was 59.4°C.

### Example 5

A 450 g portion of an aqueous lactitol solution having a concentration of 70.0% (lactitol purity, 99.8%) was cooled gradually starting from 60°C, and 0.5 g of the seed crystal (obtained by the method in Example 2) was added at a temperature of 10°C. The mixture was cooled under stirring for 20 hours to give a crystal mass.

The crystal mass was centrifuged to give 92 g (wet weight) of a crystal and 351 g of a filtrate having a concentration of 54.2%.

The crystalline substance thus obtained was dried at a temperature of 20°C for 18 hours by a cold air dehumidifying dryer to give the crystalline trihydrate lactitol of the present invention.

Measurement of the melting point and the maximum endotherm by DSC gave 56.0°C and 64.2°C, respectively.

### Example 6

A 200 g portion of an aqueous lactitol solution having a lactitol purity of 99.0% and a concentration of 83.0% was cooled gradually starting from 80°C to 15°C and poured into a stainless tray, 10 g of the crystalline mixture solid obtained by the method in Example 2 was added and was dispersed thoroughly. The mixture was left standing at room temperature over night to give massive crystals in the form of blocks.

The massive crystals in the form of blocks were ground to give a powder of the solid crystalline mixture containing crystalline trihydrate of lactitol according to the present invention.

The measurement of the melting point and maximum endotherm by DSC gave 55.0°C and 60.0°C, respectively.

### Example 7

A 100 parts by weight portion of the solid crystalline mixture containing crystalline trihydrate of lactitol obtained by the method in Example 3, and 1 part by weight of saccharin were mixed homogeneously, and a small amount of an aqueous lactitol solution having a concentration of about 75% was sprayed to give a moisture to the mixture. The mixture was then placed in a moulder for cubic sugar, pressure moulded and removed from the mould to give a solid sweetener composition which was formed in a shape of a cube.

The composition is a sweetener having a sweetness of about 1.8 times based on sugar, with substantially no hygroscopicity, being palatable and possessing a good shelf life, and thus it may be used as a low calorie sweetener which is not cariogenic.

### Example 8

Forty parts by weight of cacao paste, 10 parts by weight of cacao butter, 50 parts by weight of the trihydrate crystalline of lactitol obtained by the method in Example 2, 0.5 part by weight of aspartame and 0.5 part by weight of lecithin were mixed, and the mixture was ground into fine powder in a refiner, then placed in a conche and kneaded at a temperature of 50°C for 24 hours.

Next the mixture was placed into a mould under cooling to 31°C and solidified at 10°C.

The product is not hygroscopic and thus is useful as a low cariogenic chocolate having a good quality of sweetness.

### Example 9

Fifty parts by weight of aspirin, 15 parts by weight of the powder of the solid crystalline mixture containing crystalline trihydrate of lactitol obtained by the method in Example 3, and 4 parts by weight of corn starch were mixed thoroughly. Tablets having a thickness of 5 mm and a diameter of 6 mm were produced with a tablet machine.

The tablet is non-hygroscopic, has sufficient physical strength and a good disintegrating ability in water.

### Example 10

Ninety eight parts of the solid crystalline mixture containing crystalline trihydrate of lactitol obtained by the method in Example 1 were heated to 70°C for 5 minutes, and 1 part of malic acid, 0.25 part of cherry perfume and 0.4 art of a colouring matter were added with stirring. The mixture was then formed into a cherry candy.

The candy was well flavoured and the odour of the cherry perfume was not impaired.

## Claims

1. Crystalline trihydrate of lactitol having the molecular formula C₁₂H₂₄O₁₁.3H₂O and a melting point of 52-56°C.

2. A solid crystalline mixture containing crystalline trihydrate lactitol according to claim 1.

3. Crystalline trihydrate of lactitol and a solid crystalline mixture containing it according to either of claims 1 and 2, wherein said crystalline trihydrate of lactitol has a lactitol purity of at least 95% by weight based on the dry solid.

4. A composition containing crystalline trihydrate of lactitol or a solid crystalline mixture containing it, according to any of claims 1 to 3, together with one or more sweeteners, fillers, binders, disintegrating agents, flavourings, food dyes, vitamins, minerals or micro-organisms.

5. A composition according to claim 4 which contains one or more of starch syrup solid, glucose, maltose, high fructose corn syrup, sucrose, honey, maple syrup, sorbitol, dihydrocharcone, stevioside, α-glycosyl stevioside, glycyrrhizin, saccharin, aspartame, glycine and alanine.

6. A moulded product containing crystalline trihydrate of lactitol or a solid crystalline mixture containing it as claimed in any one of claims 1 to 5.

7. A process for the production of crystalline trihydrate of lactitol or a solid crystalline mixture containing it as claimed in claim 1 comprising crystallizing said trihydrate from an aqueous lactitol solution and collecting the crystals.

8. A process as claimed in claim 7 wherein said lactitol solution has a lactitol purity of at least 95% by weight based on dry solid.

9. A process as claimed in either of claims 7 and 8 wherein the crystallisation temperature is in the range of 0-30°C.

10. A process as claimed in any of claims 7 to 9 wherein a seed crystal is also present in said lactitol solution.

11. A process as claimed in any of claims 7 to 10 wherein an organic solvent is also present.

12. Use of crystalline trihydrate of lactitol and a solid crystalline mixture containing it as claimed in any of claims 1 to 6, for the production or manufacture of foods, drinks, confectionaries, cosmetics, pharmaceutical products, chemical materials or anti-adhesion agents.

13. Use of crystalline trihydrate of lactitol and a solid crystalline mixture containing it as claimed in any of claims 1 to 6 in low calorie foods, dietary foods, low cariogenic foods or health foods.

## Patentansprüche

1. Kristallines Trihydrat von Lactit mit der Molekülformel C₁₂H₂₄O₁₁.3H₂O und einem Schmelzpunkt von 52-56°C.

2. Eine feste kristalline Mischung, die kristallines Trihydrat von Lactit gemäß Anspruch 1 enthält.

3. Kristallines Trihydrat von Lactit und eine dieses enthaltende feste kristalline Mischung nach einem der Ansprüche 1 und 2, wobei besagtes kristallines Trihydrat von Lactit eine Lactitreinheit von wenigstens 95 Gew.-%, bezogen auf die Trockensubstanz, besitzt.

4. Eine Zusammensetzung, die kristallines Trihydrat von Lactit oder eine dieses enthaltende feste kristalline Mischung enthält, nach einem der Ansprüche 1 bis 3, zusammen mit einem oder mehreren Süßungsmitteln, Füllstoffen, Bindemitteln, Desintegrationsmitteln, Geschmacksstoffen, Lebensmittelfarbstoffen, Vitaminen, Mineralien oder Mikroorganismen.

5. Eine Zusammensetzung nach Anspruch 4, die eines oder mehreres von Maisstärkesirupfeststoff, Glucose, Maltose, Maisstärkesirup mit hohem Fructoseanteil, Saccharose, Honig, Ahornsirup, Sorbit, Dihydrochalcon, Steviosid, α-Glykosylsteviosid, Glycyrrhizin, Saccharin, Aspartame, Glycin und Alanin enthält.

6. Ein Formprodukt, das kristallines Trihydrat von Lactit oder eine dieses enthaltende feste kristalline Mischung enthält, wie beansprucht in einem der Ansprüche 1 bis 5.

7. Ein Verfahren zur Herstellung von kristallinem Trihydrat von Lactit oder einer dieses enthaltenden festen kristallinen Mischung, wie beansprucht in Anspruch 1, welches umfaßt, daß besagtes Trihydrat aus einer wäßrigen Lactitlösung auskristallisiert und die Kristalle gesammelt werden.

8. Ein Verfahren, wie beansprucht in Anspruch 7, wobei besagte Lactitlösung eine Lactitreinheit von wenigstens 95 Gew.-%, bezogen auf die Trockensubstanz, besitzt.

9. Ein Verfahren, wie beansprucht in einem der Ansprüche 7 und 8, wobei die Kristallisationstemperatur im Bereich von 0-30°C liegt.

10. Ein Verfahren, wie beansprucht in einem der Ansprüche 7 bis 9, wobei in besagter Lactitlösung auch ein Impfkristall vorhanden ist.

11. Ein Verfahren, wie beansprucht in einem der Ansprüche 7 bis 10, wobei auch ein organisches Lösungsmittel vorhanden ist.

12. Verwendung eines kristallinen Trihydrats von Lactit und einer dieses enthaltenden festen kristallinen Mischung, wie beansprucht in einem der Ansprüche 1 bis 6, für die Produktion oder Herstellung von Lebensmitteln, Getränken, Süßwaren, Kosmetika, pharmazeutischen Produkten, chemischen Materialien oder Antihaftmitteln.

13. Verwendung von kristallinem Trihydrat von Lactit und einer dieses enthaltenden festen kristallinen Mischung, wie beansprucht in einem der Ansprüche 1 bis 6, in kalorienarmen Lebensmitteln, diätetischen Lebensmitteln, wenig kariogenen Lebensmitteln oder Gesundheitslebensmitteln.

## Revendications

1. Trihydrate cristallin de lactitol de formule brute C₁₂H₂₄.3H₂O et ayant un point de fusion de 52-56°C.

2. Mélange cristallin solide contenant le trihydrate cristallin de lactitol conforme à la revendication 1.

3. Trihydrate cristallin de lactitol et mélange cristallin solide le contenant conformes à une des revendications 1 ou 2, dans lesquels le trihydrate de lactitol a une pureté en lactitol d'au moins 95 % en poids par rapport au solide sec.

4. Composition contenant du trihydrate cristallin de lactitol ou un mélange cristallin solide le contenant conformes aux revendications 1 à 3, ensemble avec un ou plusieurs agents sucrants, charges, liants, désintégrants, arômes, colorants alimentaires, vitamines, minéraux ou microorganismes.

5. Composition conforme à la revendication 4, contenant un ou plusieurs des additifs suivants : sirop d'amidon solide, glucose, maltose, sirop de maïs à haute teneur en fructose, saccharose, miel, sirop d'érable, sorbitol, dihydrochalcone, stévioside, α-glycosyl-stévioside, glycyrrhizine, saccharine, aspartame, glycine et alanine.

6. Produit moulé contenant du trihydrate cristallin de lactitol ou un mélange cristallin solide le contenant, conformes à une des revendications 1 à 5.

7. Procédé de préparation d'un trihydrate cristallin de lactitol ou d'un mélange cristallin solide le contenant, conformes à la revendication 1, comprenant la cristallisation dudit trihydrate à partir d'une solution aqueuse de lactitol et le recueillement des cristaux.

8. Procédé conforme à la revendication 7, dans lequel ladite solution de lactitol a une pureté en lactitol d'au moins 95 % en poids par rapport au solide sec.

9. Procédé conforme à une des revendications 7 ou 8, dans lequel la température de cristallisation est comprise entre 0 et 30 °C.

10. Procédé conforme à une des revendications 7 à 9 dans lequel un germe de cristallisation est également présent dans ladite solution de lactitol.

11. Procédé conforme à une des revendications 7 à 10, dans lequel est également présent un solvant organique.

12. Utilisation du trihydrate cristallin de lactitol et du mélange cristallin solide le contenant, conformes à une des revendications 1 à 6, pour la production ou la manufacture d'aliments, de boissons, de confiseries, de cosmétiques, de produits pharmaceutiques, de produits chimiques ou d'agents antiadhésifs.

13. Utilisation du trihydrate cristallin de lactitol et du mélange cristallin solide le contenant, conformes à une des revendications 1 à 16, dans des aliments à faible teneur en calories, des aliments de régime, des aliments faiblement cariogènes ou des aliments diététiques.
